Europäisches Patentamt

European Patent Office

Office européen des brevets

(11) Numéro de publication: **0 150 779**
**B1**

## (12) FASCICULE DE BREVET EUROPEEN

(45) Date de publication du fascicule du brevet: **12.10.88**

(21) Numéro de dépôt: **85100489.5**

(22) Date de dépôt: **18.01.85**

(51) Int. Cl.⁴: **A 61 F 9/00,** A 61 B 3/00

(54) Verre de contact pour l'observation et le traitement de l'oeil par irradiation lumineuse.

(30) Priorité: **30.01.84 FR 8401528**

(43) Date de publication de la demande:
**07.08.85 Bulletin 85/32**

(45) Mention de la délivrance du brevet:
**12.10.88 Bulletin 88/41**

(84) Etats contractants désignés:
**AT CH DE GB IT LI SE**

(56) Documents cités:
**EP-A-0 059 159**
**EP-A-0 092 513**
**FR-A-1 175 511**
**FR-A-2 304 315**
**GB-A- 12 885**

(73) Titulaire: **LASAG AG**
**Steffisburgstrasse 1**
**CH-3600 Thun (CH)**

(72) Inventeur: **Rol, Pascal**
**Résidence du Lac Staatsrasse 73**
**CH-3654 Gunten (CH)**

(74) Mandataire: **Gresset, Jean et al**
**ICB Ingénieurs Conseils en Brevets SA Passage**
**Max. Meuron 6**
**CH-2001 Neuchâtel (CH)**

Courier Press, Leamington Spa, England.

## Description

La présente invention concerne un verre de contact pour l'observation de l'intérieur de l'oeil et son traitement par irradiation, principalement au niveau de la rétine.

L'oeil peut être le siège de différentes maladies atteignant, entre autres, le diaphragme, le cristallin ou la rétine. Pour diagnostiquer l'affection, l'intérieur de l'oeil doit être inspecté optiquement de façon précise.

Diverses méthodes d'observation sont connues. Les images les meilleures sont obtenues lorsqu'il est fait usage d'un verre de contact appliqué sur l'oeil, permettant de déformer le moins possible le faisceau de lumière issu du point observé ou point de travail, situé à l'intérieur de l'oeil. Comme exemple on peut citer les verres de contact de Koeppe et de Goldmann, décrits en particulier dans la publication intitulée "Gonioscopie und Goniofotographie" de Winfried Müller et Hans-Peter Brandt, édition Ferdinand Enke, Stuttgart 1979.

Le verre de contact de Koeppe présente pour le faisceau lumineux une face d'entrée bombée et une face de sortie sensiblement sphérique destinée à être appliquée sur la cornée. Le verre de Goldmann, de son côté, est destiné plus particulièrement à l'observation de la rétine. Il présente au faisceau lumineux une face d'entrée plane et une face de sortie sphérique. Il peut aussi comporter une ou plusieurs faces ou parois réfléchissantes constituant des miroirs afin de permettre une observation indirecte de l'intérieur de l'eoil.

Les médecins ophtalmologues apprécient beaucoup ces deux verres de contact pour l'observation, surtout le second, étant donné leur maniabilité et le confort d'utilisation qu'ils apportent. Cependant la qualité de l'image qu'ils procurent se dégrade au fur et à mesure que l'on s'éloigne de l'axe optique de l'oeil.

Si le verre de contact est utile à l'observation de l'intérieur de l'oeil, il devient indispensable pour le traitement par irradiation de la rétine. Mais, travaillant alors dans des conditions différentes de celles de l'observation, il doit répondre à des exigences plus sévères.

Le traitement de l'intérieur de l'oeil par irradiation est en effet obtenu, en focalisant sur la région malade ou une partie de cette région, un intense faisceau de lumière cohérente. Ce faisceau peut être fourni par un laser Nd-YAG ou Argon par exemple.

Pour rendre le traitement efficace et sans danger, il est nécessaire que le faisceau atteignant le point de travail soit très bien focalisé et fortement convergent ou ouvert, c'est-à-dire que l'angle formé par les rayons lumineux extrêmes soit grand. La condition de bonne focalisation est évidente car elle permet de localiser avec précision le point de travail et d'y concentrer une énergie importante. La condition de forte convergence renforce la précédente. En effet, elle permet de bien localiser le point de travail en profondeur. D'autre part, la densité d'énergie du faisceau diminue alors rapidement en dehors du point de travail, ce qui a pour conséquence de réduire le danger le lésion des parties saines de l'oeil. La focalisation du faisceau lumineux sera d'autant meilleure que les défauts d'aberration sphérique et d'astigmatisme du système optique qu'il traverse, constitués par le verre de contact et l'oeil, seront faibles. Or généralement ces défauts augmentent avec le diamètre ou la convergence du faisceau incident. Ces deux conditions sont donc contradictoires et un compromis doit être trouvé pour chaque utilisation particulière. Le verre de Goldmann ne résoud pas de façon satisfaisante le problème lié à l'ouverture du faisceau lumineux· et encore moins celui qui concerne l'astigmatisme. Ainsi, si ce verre peut encore convenir à l'observation de la rétine, il ne permet son traitement par irradiation, dans des conditions satisfaisantes, qu'en des points situés près de l'axe de l'oeil. Pour les autres points de la rétine, les difficultés augmentent à mesure qu'ils s'éloignent de cet axe.

Diverses améliorations ont été apportées au verre de Goldmann. Des versions plus performantes de ce verre sont ainsi connues. Par exemple, le verre d'Abraham est constitué par un verre de Goldmann sans miroir sur la face d'entrée duquel est fixée, de manière décentrée, une lentille plan-convexe en verre minéral. Cette modification permet d'accroître l'angle d'ouverture du faisceau mais, en contrepartie, les aberrations ont tendance à augmenter. Un autre exemple est donné par le verre de Roussel, développé plus récemment et décrit dans la demande de brevet européen EP—A—0 059 159 au nom de la demanderesse. Ce verre est caractérisé en ce que la face de la lentille qui reçoit le faisceau lumineux constitue une surface d'onde pour ce faisceau. Il en résulte une diminution des aberrations, mais l'ouverture du faisceau n'est pas modifiée.

Il faut aussi relever que toutes les tentatives d'amélioration du verre de Goldmann ont été faites en considérant le verre de contact pour lui-même, sans tenir compte des propriétés optiques de l'oeil qu'il était destiné à observer ou à irradier. Ainsi, même un verre de contact sans défaut ne permettrait pas de focaliser parfaitement un faisceau lumineux en un point de la rétine éloigné de l'axe de l'oeil. En effet, pour atteindre ce point, le faisceau doit traverser les différentes parties de l'oeil et, en particulier, le cristallin qui présente un défaut d'astigmatisme d'autant plus grand qu'il est vu sous un angle d'incidence élevé. Ceci a pour effet de détériorer le faisceau. Or, aucun verre de contact, du type Goldmann ou autre, ne tient compte de ce phénomène, ce qui représente un défaut très gênant des verres connus destinés au traitement de la rétine en des points situés hors de l'axe de l'oeil.

L'objet principal de l'invention est de fournir un verre de contact destiné à l'examen et au traitement par irradiation des différentes parties internes de l'oeil principalement de la rétine, ne présentant pas ces inconvénients.

Pour atteindre cet objectif, le verre de contact selon l'invention pour l'observation et le traitement de l'oeil par un faisceau lumineux incident, le faisceau parcourant une distance e à l'intérieur du verre entre une face d'entrée et une face de sortie destinée à être appliquée sur l'oeil, et l'oeil présentant pour le faisceau lumineux incident un défaut d'astigmatisme défini par une distance focale sagittale S* et une distance focale tangentielle T, comprend:

—un élément principal ayant pour fonction de focaliser le faisceau lumineux incident en un point de travail à l'intérieur de l'oeil, cet élément présentant un indice de réfraction n et comportant une face d'entrée plane et une face de sortie formant la face de sortie du verre de contact, l'axe du faisceau lumineux incident atteignant la face d'entrée du verre étant déplacé par rapport à l'axe optique de l'élément principal et dirigé perpendiculairement à la face d'entrée plane,

et est caractérisé en ce qu'il comprend en outre:

—un élément de compensation présentant l'indice de réfraction n et comportant une face de sortie appliquée sur la face d'entrée plane de l'élément principal, et une face d'entrée formant la face d'entrée du verre de contact, le plan tangent à la face d'entrée de l'élément de compensation, au point où cette face est traversée par l'axe du faisceau lumineux incident, faisant un angle, avec la face d'entrée plane de l'élément principal, qui est déterminé, en fonction de e, S*, T et n, de manière que le verre de contact présente, pour le faisceau lumineux incident, un défaut d'astigmatisme inverse de celui de l'oeil.

Un avantage du verre de contact selon l'invention est de permettre la focalisation précise d'un faisceau lumineux incident en un point de la rétine, malgré les défauts optiques des parties de l'oeil traversées par ce faisceau.

D'autres avantages ressortiront de la description qui sera faite maintenant à l'aide du dessin annexé qui illustre, à titre d'exemple nullement limitatif, un verre de contact selon l'invention. Sur ce dessin:

—la figure 1 montre, en coupe selon un plan de symétrie longitudinal, une représentation schématique de l'oeil sur la cornée duquel est appliqué un verre de contact de Goldmann déjà cité; et

—les figures 2a et 2d représentent des exemples de réalisation du verre de contact selon l'invention.

Les mêmes éléments portent sur toutes les figures les mêmes références.

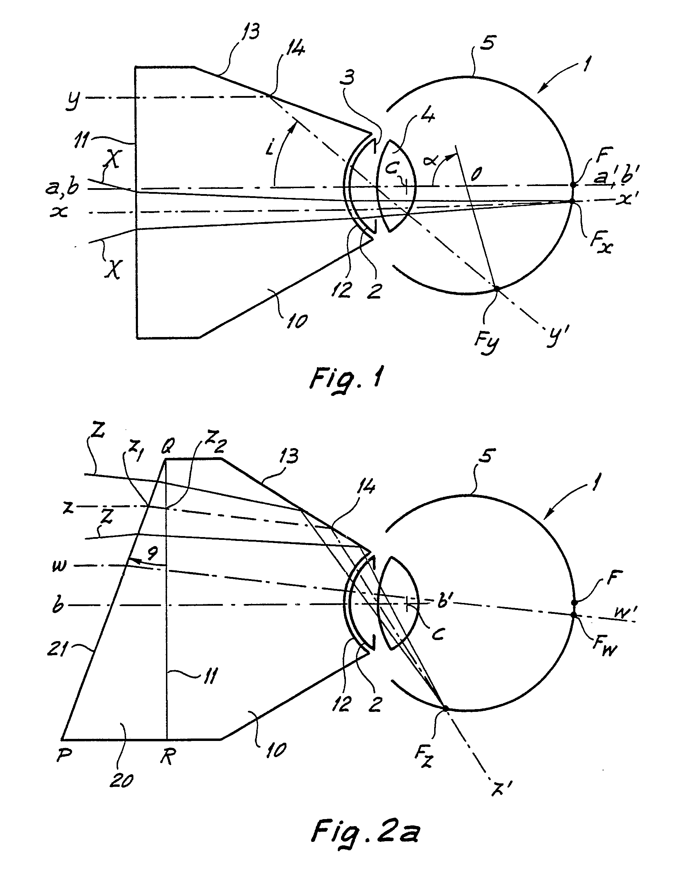

Une représentation schématique de l'oeil 1 est montrée sur la figure 1. C'est un corps de révolution ayant un axe de symétrie aa' qui comprend, en allant de l'extérieur vers l'intérieur de l'oeil, une cornée 2 par laquelle entrent les rayons lumineux, un iris 3 dont l'ouverture règle la quantité de lumière, un cristallin 4 et une rétine 5, de forme sphérique ayant un centre 0, qui est traversée par l'axe aa' en un point F. Pour bien comprendre la présente invention, il est utile de décrire d'abord la structure du verre de contact de Goldmann à miroir, déjà connu de l'art antérieur, et aussi représenté sur la figure 1. Ce verre, référencé 10, comprend une face d'entrée plane 11, une face de sortie sphérique 12 appliquée sur la cornée 2, et une face réfléchissante 13. D'autres faces réfléchissantes, non représentées, sont réparties sur le pourtour du verre. L'angle entre une face réfléchissante et la face d'entrée dépend du point de la rétine visée. Cet angle peut varier de 50° à 80°. La droite perpendiculaire à la face d'entrée 11 et passant par le centre de courbure C de la face de sortie 12 définit l'axe de symétrie bb' du verre de contact 10. Dans le cas de la figure 1, les axes aa' et bb' sont confondus. Les dimensions du verre 10 permettent son basculement sur la cornée 2 de façon à pourvoir déplacer, dans certaines limites, le point d'observation ou de traitement, dit aussi point de travail, à l'intérieur de l'oeil. L'axe bb' pivote alors autour du centre C.

Le domaine d'application du verre de Goldmann est assez étendu puisque pratiquement tous les points essentiels de l'intérieur de l'oeil lui sont accessibles, aussi bien pour l'observation que pour le traitement. Si l'efficacité de ce verre est bonne pour les points situés près de la cornée 2, elle est par contre médiocre pour ceux situés sur la rétine 5 loin de l'axe aa' pour des raisons qui seront données par la suite.

Le but de la présente invention étant de fournir un verre de Goldmann modifié qui permette d'observer et surtout de traiter par irradiation tout point de la rétine 5 dans de bonnes conditions, la figure 1 montre le cheminement de la lumière dans un verre de Goldmann pour deux points typiques de la rétine. Le premier point, noté $F_x$ sur la figure 1, correspond à un point voisin de l'axe aa' de l'oeil. Pour atteindre ce point, un faisceau de lumière incident X, d'axe xx', doit entrer dans le verre de Goldmann 10 perpendiculairement à la face d'entrée 11, à une certaine distance de l'axe aa', et traverser directement, sans réflexion, le verre 10 et l'oeil 1. Le second point, noté $F_y$, correspond à un point situé loin de l'axe aa'. L'angle α formé par l'axe aa' et la droite joignant le point $F_y$ au centre 0 de la rétine 5 peut dépasser 90°. Pour atteindre le point $F_y$, un faisceau de lumière Y, dont seul l'axe yy' a été représenté, doit, après sont entrée dans le verre 10 perpendiculairement à la face 11, subir une réflexion totale par la face 13 en un point 14 afin de pénétrer dans l'oeil 1 sous un angle d'incidence i. Pour chaque région de la rétine 5 éloignée de l'axe aa', correspond une inclinaison particulière de la face 13 par rapport à la face 11 du verre 10. C'est pourquoi, un même verre de Goldmann peut présenter avantageusement plusieurs faces réfléchissantes d'inclinaisons différentes.

L'oeil n'étant pas un système optique parfait, même un verre de contact sans défaut ne permettra pas de focaliser un faisceau lumineux en un point, mais seulement au mieux dans un cercle de diffusion de diamètre plus ou moins réduit. En effet, l'oeil présente un défaut d'astigmatisme d'autant plus prononcé que l'angle d'incidence i du faisceau lumineux traversant le cristallin est élevé. Pour obtenir une focalisation précise, le verre de contact doit donc présenter un défaut d'astigmatisme inverse de celui de l'oeil.

Il existe plusieurs modèles de l'oeil, par exemple celui de Gullstrand-Legrand ou celui de Littmann, permettant de décrire avec précision ses propriétés optiques. Il est ainsi bien connu que l'oeil, comme toute surface optique sphérique, présente un défaut d'astigmatisme qui peut être décomposé en une partie sagittale et une partie tangentielle. Chaque partie est définie par sa distance focale, mesurée, par exemple, à partir de la face d'entrée de l'oeil. La distance focale correspondant à la partie sagittale est désignée par S* et celle correspondant à la partie tangentielle par T.

Des considérations théoriques et des essais ont montré qu'il est possible d'obtenir un verre de contact compensé présentant le défaut d'astigmatisme inverse voulu en associant à la face d'entrée d'un verre de Goldmann un élément de compensation de forme simple, facile et peu coûteux à réaliser.

L'élément de compensation modifie le point de visée du verre de Goldmann. Cette modification est cependant faible et elle peut être compensée par un léger changement, de quelques degrés, de l'inclinaison de la face réfléchissante. Pratiquement, il est tourjours possible de fabriquer ou de trouver, pour chaque point de visée, un verre du type de Goldmann adéquat. En effet, il existe des verres standard dont l'inclinaison de la face réfléchissante, par rapport à la face d'entrée, est comprise entre 50° et 80°.

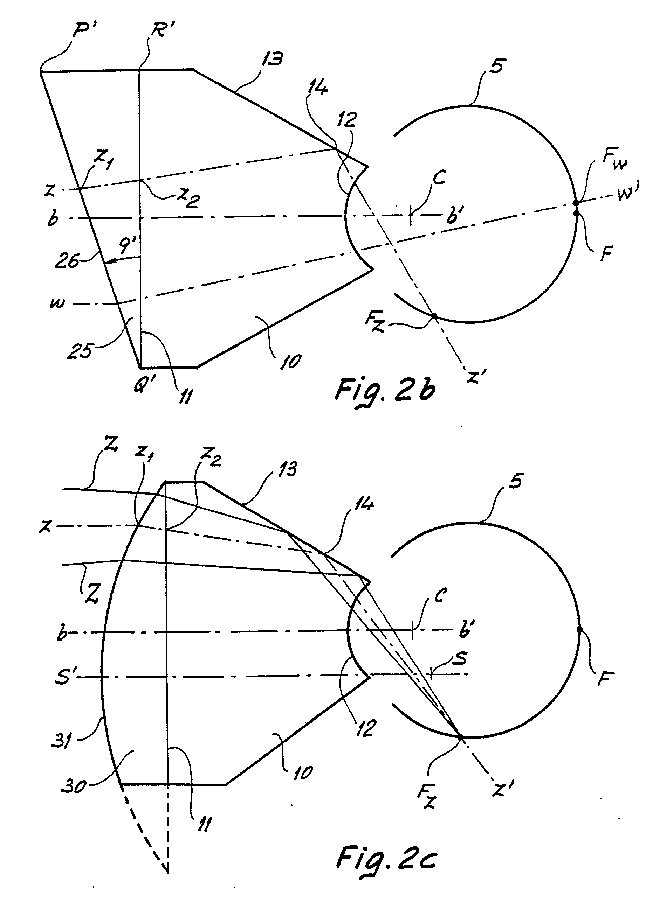

Une première forme d'exécution d'un verre de contact compensé selon l'invention est représentée sur la figure 2a en coupe longitudinale selon un plan de symétrie du verre. Dans cet exemple, l'élément de compensation est un primse 20 dont l'intersection avec le plan de la figure est un triangle de sommets PQR, rectangle en R, dont les arêtes sont perpendiculaires au plan du dessin. Ce prisme est orienté de façon que, d'une part, sa face contenant les points Q et R soit appliquée sur la face d'entrée 11 du verre de Goldmann 10 et que, d'autre part, le somme Q se trouve du même côté, par rapport à l'axe bb', que la face réfléchissante 13. Le plan du prisme 20 contenant les points P et Q devient ainsi la face d'entrée 21 du verre de contact compensé.

Sur la figure 2a est également représenté le trajet d'un faisceau lumineux Z, d'axe zz' en supposant que le verre de Goldmann 10 et le prisme 20 sont réalisés dans le même matériau. La direction du faisceau incident est choisie parallèle à l'axe bb' du verre de contact. L'axe zz' du faisceau pénètre dans le prisme 20 obliquement à la face 21, en un point $z_1$ de cette face. L'indice de réfraction n du prisme (de l'ordre de 1.5) étant plus élevé que celui de l'air (égal à 1), les rayons lumineux du faisceau Z subissent une déviation par réfraction en traversant la face 21. Après cette réfraction, l'axe zz' traverse la face d'entrée 11 du verre de Goldmann 10 aussi obliquement, en un point $z_2$. Cette fois, le faisceau ne subit cependant pas de réfraction, les deux milieux de part et d'autre de la face 11 étant supposés être indentiques. L'axe zz' atteint alors un point 14 de la face 13 où il subit une réflexion totale, puis il pènètre dans l'oeil 1 et atteint la rétine 5 en un point $F_z$.

Dans la forme d'exécution du verre de contact composé représentée sur la figure 2a, l'élément de compensation est le prisme 20. Or il est bien connu qu'un tel prisme présente, pour le faisceau lumineux Z d'axe zz', un défaut d'astigmatisme. Cet astigmatisme dépend de l'angle au somme Q du triangle PQR, désigné par q, et de la distance e, non indiquée sur le dessin, que parcourent les rayons lumineux entre la face d'entrée 21 et la face de sortie 12 du verre de contact ayant un indice de réfraction n.

Si $q = \arcsin[(T - S*)/(T + e - (S* + e)/n^2)]^{1/2}$,

S* et T étant, comme déjà mentionné, les distances focales correspondant respectivement aux parties sagittale et tangentielle de l'astigmatisme, alors celui du prisme devient exactement inverse de celui de l'oeil, réalisant ainsi la correction voulue du verre de Goldmann. Si le matériau utilisé est un plastique pour lequel n est égal à 1,5, l'angle q vaut environ 20° pour le point $F_z$ visé de la figure 2a.

Le point $z_1$ de l'axe zz' est situé entre le point Q et l'axe bb', sur la face 21. La longueur e du trajet des rayons lumineux dans le verre de contact dépendant du point $z_1$, à chaque position de ce point comprend une valeur bien déterminée de l'angle q. Cette valeur permet d'obtenir la meilleure focalisation possible du faisceau lumineux incident Z au point $F_z$ de la rétine 5.

Bien entendu, le verre de contact corrigé de la figure 2a peut être fait d'une seule pièce. D'autre part, il est évident que seule la partie du prisme 20 qui reçoit le faisceau de lumière Z est fonctionnelle. Le reste du prisme peut avoir une forme quelconque. En particulier, l'angle au sommet R du triangle de la base du prisme peut être différent de 90°.

Une autre forme d'exécution, utilisant un prisme 25 comme élément de compensation, est représentée sur la figure 2b. Cette nouvelle forme d'exécution est semblable à celle représentée sur la figure 2a, avec la seule différence que le prisme 25, ayant une base triangulaire P'Q'R', rectangle en R', a une orientation telle que les sommets P' et R' se trouvent du même côté, par rapport à l'axe bb', que la face réfléchissante 13 du verre de Goldmann 10. La position du prisme 25 sur le verre de Goldmann est donc obtenue par une rotation de 180° du prisme 20 autour d'un axe parallèle à l'axe bb'.

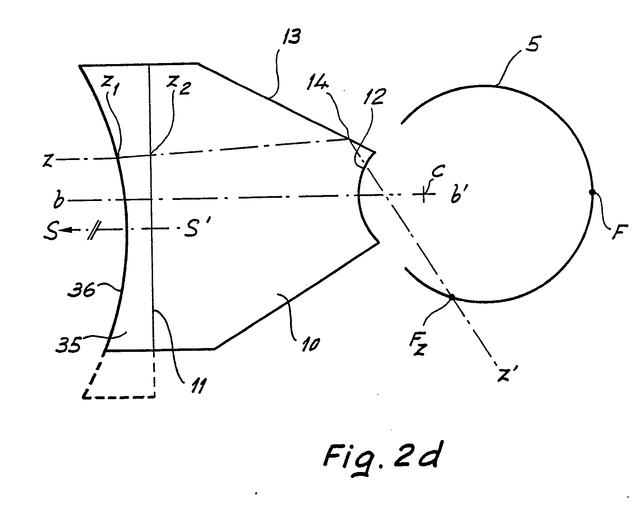

Le prisme 25 a une face d'entrée 26 sur laquelle tombe un faisceau lumineux d'axe zz'. Cet axe passe par les points $z_1$, $z_2$ et 14 pour atteindre la rétine 5 au point $F_z$, comme dans le cas de la figure 2a. L'orientation différente du prisme de compensation fait cependant que le point $F_z$ est légèrement plus éloigné du point F de l'oeil 1 que dans le cas de la figure 2a si l'on garde le même angle pour le miroir 13. Une autre inclinaison de ce miroir permet, comme dans la figure 2b, d'atteindre le point $F_z$ de la figure 2a.

Pour que le prisme 25 présente un défaut d'astigmatisme exactement inverse de celui de l'oeil, l'angle q' au sommet Q' du triangle P'Q'R' doit avoir une valeur bien définie, donnée par la même relation que celle écrite pour l'angle q.

Les verres de contact des figures 2a et 2b peuvent également être utilisés en transparence avec des angles q ou q' beaucoup plus faibles mais toujours donnés par la même relation que précédemment. Dans ce mode d'utilisation, un faisceau lumineux d'axe ww' traverse le verre de contact sand être réfléchi par la face 13 du verre de Goldmann. Il permet d'atteindre alors un point $F_w$ de la rétine, situé très près de l'axe de l'oeil. Le faisceau lumineux d'axe ww' rencontre les faces d'entrée 21 ou 26, visibles sur les figures 2a et 2b, sous le même angle que le faisceau d'axe zz' et, dans le verre de contact, il suit un chemin parallèle à la partie de l'axe zz' qui joint les points $z_1$ et 14.

Une seconde forme d'exécution d'un verre de contact compensé est représentée sur la figure 2c. L'élément de compensation est, dans ce cas, au moins une portion d'une lentille plan-convexe 30 ayant une face plane et une face sphérique 31. La partie plane de la lentille est mise en contact avec la face d'entrée 11 du verre de Goldmann. La partie sphérique 31 de la lentille constitue la face d'entrée du verre de contact. Cette face est définie par la position de son centre de courbure S, par rapport au verre de Goldmann 10, et par son rayon de courbure. La droite, perpendiculaire à la partie plane de la lentille et passant par le centre S, définit l'axe SS' de la lentille. Cet axe est parallèle à l'axe bb'. La lentille 30 peut être tronquée en certains endroits de son contour, comme cela est montré en pointillé sur la figure 2c.

Pour un faisceau incident Z, d'axe zz' parallèle à l'axe bb', des formules d'optique, connues sous le nom d'équations de Coddington, figurant par exemple aux pages 186 et 187 de l'ouvrage "Lens Design Foudamentals" de Rudolph Kingslake, Academic Press, New-York 1978, permettent de calculer le rayon de courbure de la face 31 et la distance entre les axes SS' et zz', laquelle permet de déterminer la distance entre les axes SS' et bb'. Plusieurs solutions sont possibles. Si le matériau est du plastique, ces deux grandeurs peuvent valoir, par exemple, respectivement 42 mm et 8 mm, le point S étant situé du côté opposé à la face 13, par rapport à l'axe bb'.

Un faisceau lumineux incident Z, d'axe zz', arrivant sur le verre de contact parallèlement à l'axe bb', est réfracté en traversant la face d'entrée 31 puis réfléchi par la face 13 de façon à parvenir au point $F_z$ de la rétine 5, situé, dans ce cas, loin de l'axe de l'oeil ou du point F.

Une autre variante utilisant une lentille comme élément de compensation est représentée sur la figure 2d. Au moins une portion d'une lentille plan-concave 35, ayant une face plane et une face sphérique 36, est employée dans ce cas. La partie plane de cette lentille est appliquée sur la face d'entrée 11 du verre de Goldmann 10 et sa partie sphérique 36, constituant la face d'entrée du verre de contact corrigé, est définie, comme dans le cas précédent, par son centre de courbure S, rejeté en dehors de la figure, et par son rayon de courbure. Si le même matériau plastique est utilisé pour la lentille de compensation et pour le verre de Goldmann, les équations de Coddington montrent que le centre de courbure S de la lentille doit être situé approximativement, par exemple à 2 mm de l'axe bb', du côté opposé à celui de la face 13, et son rayon de courbure doit être égal à environ 75 mm.

Le cheminement d'un faisceau lumineux incident, représenté par son axe zz', est également montré sur la figure 2d. Le faisceau arrive sur la face 36 parallèlement à l'axe bb' puis, après une réfraction au point $z_1$ et une réflexion au point 14 sur la face 13, inclinée convenablement, il atteint la rétine 5 au point $F_z$.

L'élément de compensation et le verre de Goldmann étant réalisés dans le même matériau peuvent être réalisés sous forme de deux composants distincts. Ces deux composants, une fois réunis, permettent de définir physiquement une face 11 dans le verre de contact ainsi obtenu. Cette face n'a cependant aucun effet sur les rayons lumineux, les indices de réfraction des deux côtés de cette face étant les mêmes. Le verre de contact compensé peut donc, tout aussi bien, être réalisé d'une seule pièce homogène dans laquelle la face 11 ne peut plus être localisée. Ce verre d'une seule pièce aura, bien entendu, les mêmes propriétés que la verre précédent.

## Revendications

1. Verre de contact pour l'observation et le traitement de l'oeil (1) par un faisceau lumineux incident (Z), ledit faisceau parcourant une distance e à l'intérieur dudit verre entre une face d'entrée (21, 26, 31, 36) et une face de sortie (12) destinée à être appliquée sur l'oeil, et ledit oeil présentant pour ledit faisceau un défaut d'astigmatisme défini par une distance focale sagittale S* et une distance focale tangentielle T, comprenant:

—un élément principal (10) ayant pour fonction de focaliser le faisceau lumineux incident (Z) en un point de travail ($F_z$) à l'intérieur de l'oeil, ledit élément présentant un indice de réfraction n et comportant une face d'entrée plane (11) et une face de sortie formant ladite face de sortie (12) du verre de contact, l'axe du faisceau lumineux incident (Z) atteignant la face d'entrée dudit verre étant déplacé par rapport à l'axe optique de l'élément principal et dirigé perpendiculairement à ladite face d'entrée plane, caractérisé en ce qu'il comprend en outre:

—un élément de compensation (20, 25, 30, 35) présentant l'indice de réfraction n et comportant une face de sortie appliquée sur la face d'entrée plane (11) de l'élément principal (10), et une face d'entrée (21, 26, 31, 36) formant ladite face d'entrée dudit verre, le plan tangent à ladite face d'entrée de l'élément de compensation, au point ($z_1$) où cette face est traversée par l'axe du faisceau lumineux incident (Z), faisant un angle (q, q'), avec la fase d'entrée plane (11) de l'élément principal, qui est déterminé, en fonction de e, S*, T et n, de manière que ledit verre de contact présente, pour ledit faisceau, un défaut d'astigmatisme inverse de celui de l'oeil.

2. Verre de contact selon la revendication 1, caractérisé en ce que l'élément principal (10) est un verre de Goldmann ayant une face d'entrée plane (11), une face réfléchissante (13) et une face de sortie (12) sphérique définissant un axe de symétrie (bb') perpendiculaire à la face d'entrée plane (11) et passant par le centre de courbure (C) de la face de sortie (12) sphérique, et en ce que ledit élément de compensation (20, 25, 30, 35) étant appliqué sur ladite d'entrée plane (11) crée dans ledit verre ledit défaut d'astigmatisme inverse pour un faisceau lumineux incident (Z) d'axe parallèle audit axe de symétrie (bb') et frappant le verre de contact du côté de ladite face réfléchissante (13).

3. Verre de contact selon la revendication 2, caractérisé en ce que ledit élément de compensation est un prisme (20, 25).

4. Verre de contact selon la revendication 2, caractérisé en ce que ledit élément de compensation est au moins une portion (30) d'une lentille plan-convexe présentant un axe de symétrie (SS').

5. Verre de contact selon la revendication 2, caractérisé en ce que ledit élément de compensation est au moins une portion (35) d'une lentille plan-concave présentant un axe de symétrie (SS').

6. Verre de contact selon l'une des revendications 4 et 5, caractérisé en ce que l'axe de symétrie (SS') dudit élément de compensation et l'axe (bb') de la face de sortie (12) du verre de Goldmann sont décalés l'un par rapport à l'autre.

7. Verre de contact selon la revendication 1, caractérisé en ce que ledit élément principal et ledit élément de compensation sont réunis en une seule pièce homogène.

8. Verre de contact selon la revendication 1, caractérisé en ce que ledit angle (q, q') est défini par la formule suivante:

$$\arcsin \left[ (T-S^*)/(T+e-(S^*+e)/n^2) \right]^{1/2}.$$

**Patentansprüche**

1. Kontaktglas für die Untersuchung und Behandlung des Auges (1) durch ein einfallendes Strahlenbündel (Z), wobei das Strahlenbündel in Inneren dieses Glases zwischen einer Eintrittsfläche (21, 26, 31, 36) und einer Austrittsfläche (12), die dazu bestimmt ist, auf das Auge aufgesetzt zu werden, eine Distanz e zurücklegt, und das Auge gegenüber dem Strahlenbündel einen Astigmatismusfehler aufweist, der durch einen sagittalen Brennpunktabstand S* und einen tangentialen Brennpunktabstand T definiert ist, welches umfaßt:

——ein Hauptelement (10), dessen Aufgabe es ist, das einfallende Strahlenbündel (Z) an einem Arbeitspunkt ($F_z$) im Augeninneren zu fokussieren, wobei dieses Element einen Refraktionskoeffizienten n aufweist und eine ebene Eintrittsfläche (11) und eine Austrittsfläche besitzt, welche die Austrittsfläche (12) des Kontaktglases bildet, wobei die Achse des auf die Eintrittsfläche auftreffenden einfallenden Strahlenbündels (Z) im Verhältnis zur optischen Achse des Hauptelementes versetzt und senkrecht zur ebenen Eintrittsfläche ausgerichtet ist,
dadurch gekennzeichnet, daß es außerdem umfaßt:
—ein Ausgleichselement (20, 25, 30, 35) mit dem Refractions koeffizienten n, das eine Austrittsfläche besitzt, welche auf der ebene Eintrittsfläche (11) des Hauptelementes (10) aufliegt, und eines Eintrittsfläche (21, 26, 31, 36), welche die Eintrittsfläche des Glases bildet, wobei die zur Eintrittsfläche des Ausgleichselementes am Punkt ($z_1$), wo diese Fläche von der Achse des einfallenden Lichtbündels (Z) durchquert wird, tangentiale Ebene einen Winkel (q, q') mit der ebenen Eintrittsfläche (11) des Hauptelementes bildet, der nach Maßgabe von e, S*, T und n so bestimmt wird, daß das Kontaktglas für das Strahlenbündel einen gegenüber jenem des Auges umgekehrten Astigmatismusfehler aufweist.

2. Kontaktglas nach Anspruch 1, dadurch gekennzeichnet, daß das Hauptelement ein Goldmannsches Glas (10) ist, welches eine ebene Eintrittsfläche (11), eine reflektierende Fläche (13) und eine sphärische Ausgangsfläche (12) besitzt, wodurch eine Symmetrieachse (bb') definiert wird, die senkrecht zur ebenen Eintrittsfläche (11) und durch den Krümmungsmittelpunkt (C) der sphärischen Ausgangsfläche (12) verläuft, und daß das auf die ebene Eintrittsfläche (11) aufgesetzte Ausgleichselement (20, 25, 30, 35) im Glas den umgekehrten Astigmatismusfehler für ein einfallendes Strahlenbündel (Z) mit einer zur Symmetrieachse (bb') parallelen Achse, das auf das Kontaktglas auf der Seite der reflektierenden Fläche (13) auftrifft, hervorruft.

3. Kontaktglas nach Anspruch 2, dadurch gekennzeichnet, daß das Ausgleichselement ein Prisma ist (20, 25).

4. Kontaktglas nach Anspruch 2, dadurch gekennzeichnet, daß das Ausgleichselement zumindest ein Teil (30) einer plankonvexen Linse mit einer Symmetrieachse (SS') ist.

5. Kontaktglas nach Anspruch 2, dadurch gekennzeichnet, daß das Ausgleichselement zumindest ein Teil (35) einer plankonkaven Linse mit einer Symmetrieachse (SS') ist.

6. Kontaktglas nach einem der Ansprüche 4 und 5, dadurch gekennzeichnet, daß die Symmetrieachse (SS') des Ausgleichselementes und die Achse (bb') der Austrittsfläche (12) des Goldmannschen Glases gegenseitig versetzt sind.

7. Kontaktglas nach Anspruch 1, dadurch gekennzeichnet, daß das Hauptelement und das Ausgleichselement in einem einzigen homogenen Stück zusammengefaßt sind.

8. Kontaktglas nach Anspruch 1, dadurch gekennzeichnet, daß der Winkel (q, q') durch nachstehende Formel definiert ist;

$$\text{arc sin}[(T-S^*)/(T+e-(S^*+e)/n^2)]^{1/2}$$

**Claims**

1. A contact lens arrangement for observing and treating an eye (1) with an incident light beam (Z), said beam travelling a distance e within said lens arrangement between an entry surface (21, 26, 31, 36) and an exit surface (12) destined to be applied to the eye, and said eye having towards said beam an astigmatism that is defined by a sagittal focal distance $S^*$ and a tangential focal distance T, comprising:
—a main element (10) for focussing the incident light beam (Z) on a working point ($F_z$) within the eye, said element having a refractive index n and having a plane entry surface (11) and an exit surface forming said exit surface (12) of the contact lens arrangement, the axis of the incident light beam (Z) reaching the entry surface of said lens arrangement being shifted in relation to the optical axis of the main element and being directed perpendicularly to said plane entry surface,
characterized in that it further comprises:
—a compensating element (20, 25, 30, 35) having said refractive index n and having an exit surface that is applied to the plane entry surface (11) of the main element (10) and an entry surface (21, 26, 31, 36) that forms said entry surface of said lens arrangement, the plane that is tangent to said entry surface of the compensating element, at the point ($z_1$) where the axis of the incident light beam (Z) passes through said surface, forming with the plane entry surface (11) of the main element an angle (q, q') that is so determined, in dependence on e, $S^*$, T and n, that said contact lens arrangement has, towards said beam, an astigmatism opposite to that of the eye.

2. A contact lens arrangement according to claim 1, characterized in that the main element (10) is a Goldmann lens having a plane entry surface (11), a reflecting surface (13) and a spherical exit surface (12) defining an axis of symmetry (bb') perpendicular to the plane entry surface (11) and passing through the centre of curvature (C) of the spherical exit surface (12), and in that said compensating element (20, 25, 30, 35), by being applied to said plane entry surface (11), produces in said lens arrangement said opposite astigmatism towards an incident light beam (Z) having an axis parallel to said axis of symmetry (bb') and which impinges on the contact lens arrangement on the side of the reflecting surface (13).

3. A contact lens arrangement according to claim 2, characterized in that said compensating element is a prism (20, 25).

4. A contact lens arrangement according to claim 2, characterized in that said compensating element is at least a portion (30) of a plano-convex lens having an axis of symmetry (SS').

5. A contact lens arrangement according to claim 2, characterized in that said compensating element is at least a portion (35) of a plano-concave lens having an axis of symmetry (SS').

6. A contact lens arrangement according to either of claims 4 and 5, characterized in that the axis of symmetry (SS') of said compensating element and the axis (bb') of the exit surface (12) of the Goldmann lens are shifted in relation to each other.

7. A contact lens arrangement according to claim 1, characterized in that said main element and said compensating element are united to form a single, homogeneous part.

8. A contact lens arrangement according to claim 1, characterized in that said angle (q, q') is defined by the following formula:

$$\text{arc sin}[(T-S^*)/(T+e-(S^*+e)/n^2)]^{1/2}.$$

Fig. 1

Fig. 2a

Fig. 2b

Fig. 2c

Fig. 2d